# FASCICULE DE BREVET EUROPEEN

(11) **EP 0 998 445 B1**
(45) Date de publication et mention de la délivrance du brevet: **01.10.2003**
(21) Numéro de dépôt: 98939682.5
(22) Date de dépôt: 15.07.1998
(51) Int. Cl.: C07C 69/017, C07C 67/14, C07C 67/08, A61K 7/48, A61K 31/215

(54) **COMPOSITIONS A BASE DE DERIVES DE RESVERATROL**
COMPOSITIONEN AUF BASIS VON RESVERATROLDERIVATEN
COMPOSITIONS BASED ON RESVERATROL

(30) Priorité: 15.07.1997 FR 9708964
(43) Date de publication de la demande: 10.05.2000
(73) Titulaire: Caudalie, 75017 Paris (FR)
(72) Inventeur: VERCAUTEREN, Joseph, F-33600 Pessac (FR); CASTAGNINO, Chantal, Résidence Club, F-33700 Merignac (FR); DELAUNAY, Jean-Claude, F-33700 Merignac (FR)
(74) Mandataire: Peaucelle, Chantal
(86) Numéro de dépôt international: FR9801548
(87) Numéro de publication internationale: WO99003816

(56) Documents cités:
- CHEMICAL ABSTRACTS, vol. 60, no. 4, 17 février 1964 Columbus, Ohio, US; abstract no. 4240c, SUSUMU NONOMURA ET AL: "Chemical constituents of polygonaceous plants.I.Components of Polygonum cuspidatum" colonne 4240; XP002058089 & SUSUMU NONOMURA TE AL.: YAKUGAKU ZASSHI, vol. 83, 1963, pages 988-990, JAPAN

## Description

L'invention concerne des compositions à base de dérivés de resvératrol présentant notamment une grande stabilité à l'air et à la lumière.

Le resvératrol (3, 5, 4'-trihydroxystilbène) existe sous la forme cis ou trans et se présente sous forme monomère, ou comme oligomère renfermant généralement 2 à 4 motifs monomères.

Dans la suite de la description et dans les revendications, on utilisera le terme "ORs" pour désigner aussi bien le monomère que les oligomères.

L'étude des propriétés du resvératrol a permis de mettre en évidence des activités biologiques d'intérêt. Des effets cardiovasculaire et anti-cancérigène ont ainsi été rapportés (M. Jang et al., Science, 275, 218-220 (1997)).

L'utilisation pratique des ORs est rendue difficile par l'accès malaisé, sélectivement, à de tels extraits, à partir des sources végétales qui les contiennent. Elle l'est également en raison de leur instabilité, due aux groupes phénoliques qu'ils renferment, et à leur caractère hydrosoluble, qui pose des problèmes de miscibilité avec de nombreux excipients utilisés généralement en thérapeutique, cosmétique et dans le domaine alimentaire, qui présentent au contraire des propriétés liposolubles.

Pour résoudre ces problèmes, les inventeurs ont mis au point des méthodes extractives conduisant à des extraits enrichis en ORs, et ont utilisé des groupements protecteurs des fonctions phénoliques, qui permettent à la fois de conférer une stabilité satisfaisante au resvératrol monomère et oligomère et de les rendre liposolubles, ces groupements présentant l'avantage d'être éliminables in vivo.

L'invention a donc pour but de fournir des compositions à base de dérivés de monomères et/ou d'oligomères de resvératrol dont les groupements protecteurs peuvent être aisément éliminés pour libérer le principe actif quand souhaité.

Elle vise également à fournir un procédé d'obtention de ces compositions ainsi que des monomères et/ou oligomères de départ.

L'invention vise en outre les applications de ces compositions dans divers domaines, notamment en thérapeutique, en cosmétique et dans l'agro-alimentaire.

Les compositions de l'invention sont caractérisées en ce qu'elles sont essentiellement à base d'esters de resvératrol monomères et/ou oligomères, les monomères comportant au moins un groupe ester de formule -O-CO-A, et les oligomères étant formés de motifs monomères réunis par des liaisons carbone-carbone, ou éther, et/ou de monomères réticulés par des groupes

-O-CO-R-CO-O-

- A représentant un radical alkyle d'au moins deux atomes de carbone, linéaire ou ramifié, saturé ou insaturé, un radical aryle, excepté le radical phényle dans le cas d'une composition de resvératrol monomère, aralkyle ou aralkylène (S. Nonomura et al., Chem. Abstr., vol. 60, n°. 4, abstract no. 4240c, 1964), et
- R représentant un radical alkylène de 0 à 10 atomes de carbone, saturé ou insaturé, et/ou 1 radical arylène ayant 1 à 3 cycles et/ou un radical hétérocyclique, et les diastéréoisomères de ces motifs.

Ces compositions peuvent être conservées sur une longue période sans altération, notamment pendant au moins 2 ans dans des conditions normales de conservation (température de 10 à 22°C, en conditionnement protégeant de la lumière, hygrométrie 40-50 %).

Dans un mode de réalisation de l'invention, les compositions sont à base de monomères et/ou d'oligomères de resvératrol comportant au moins un groupe -O-CO-A.

Dans un groupe préféré, A représente un radical d'acide gras saturé ou insaturé.

Dans le cas d'une insaturation, les doubles liaisons sont avantageusement cis, ce qui correspond au cas le plus fréquent chez les produits naturels. Avec des produits obtenus plus particulièrement par synthèse ou hémisynthèse, les liaisons sont trans.

Parmi les acides gras convenant pour la mise en oeuvre de l'invention, on citera les acides butyrique C4:O ; valérique, C5:O hexanoïque, C6:O:sorbique, C6:2(n-2) ; laurique C12:O ; palmitique C16:O ; stéarique, C18:O; oléique, C18:1(n-9) ; linoléique, C18:2(n-6) ; linolénique, C18:3(n-6) ; α linolénique, C18:3(n-3) ; arachidonique, C20:4(n-3) ; éicosapentaénoïque C20:5(n- 3); et docosahexaénoïque. C22:6(n-3).

Les acides gras en C16 et plus sont particulièrement appropriés en vue d'applications en cosmétique. Ces acides gras sont extraits, par exemple, de microalgues.

Dans un autre groupe préféré, A représente un groupe aryle, excepté, comme précisé plus haut, le radical phényle dans le cas d'une composition de resvératrol monomère.

Dans encore un autre groupe, A représente un groupe aralkyle ou aralkylène, le groupe alkyle ou alkylène étant plus particulièrement en C1 à C8, notamment en C1 à C4. On citera, notamment le groupe benzyle ou styryle.

Dans un autre mode de réalisation de l'invention, les compositions sont à base de monomères et/ou d'oligomères de resvératrol réticulés par l'intermédiaire de ponts -O-CO-R-CO-O-.

Dans cette structure, R représente un radical alkylène de 0 à 10 atomes de carbone, saturé ou insaturé, et/ou un radical arylène comportant 1 à 3 cycles et/ou un radical hétérocyclique.

Des esters réticulés avantageux comprennent, comme substituant R, un radical d'un diacide choisi parmi acide malique, malonique, glutarique, phtalique, d'un chlorure de diacides, comme le dichlorure de téréphtaloyle, le dichlorure de succinyle, le dichlorure de sébacoyle, et le dichlorure d'adipoyle, d'un anhydride, ou encore d'un isocyanate comme le diisocyanate de toluène ou d'hexaméthylène.

D'une manière avantageuse, ces compositions réticulées forment des microcapsules ou des masses spongieuses.

L'invention vise également un procédé d'obtention des esters définis ci-dessus.

Ce procédé est caractérisé en ce qu'il comprend la réaction de monomères et/ou d'oligomères de resvératrol avec, comme agents d'acylation, des composés de formule A-CO-O-A1, ou A1-O-CO-R-CO-O-A1, où
- A représente un radical alkyle d'au moins deux atomes de carbone, linéaire ou ramifié, saturé ou insaturé, un radical aryle, aralkyle ou aralkylène,
- R représente un radical alkylène de 0 à 10 atomes de carbone, saturé ou insaturé, et/ou 1 radical arylène ayant 1 à 3 cycles et/ou un radical hétérocyclique, et
- A1 représente un atome d'hydrogène, d'halogène, un radical alcoyle en C1 à C8, ou aryle, un groupe -CO-A, ou isocyanate, A et A1 ne pouvant représenter un radical phényle et un atome de chlore dans A-CO-O-A1.

Les réactions d'estérification avec des acides sont réalisées généralement à température ambiante, en présence d'un agent d'activation. On citera par exemple le dicyclohexylcarbodiimide (DCC) ou le terbutylchloroformiate.

Les estérifications avec les dérivés d'acide sont effectuées avantageusement selon la réaction de Schotten Baumann, en milieu aqueux alcalin.

Ces réactions conduisent à l'obtention des compositions estérifiées sous forme spongieuse, que l'on isole du mélange réactionnel et que l'on purifie en vue des applications ultérieures envisagées.

Lorsqu'on utilise des diacides ou leurs dérivés, on forme une émulsion de type (E/H) par dispersion, sous agitation, d'une solution aqueuse alcaline des monomères et/ou oligomères de resvératrol dans un solvant organique non miscible à l'eau, puis on ajoute l'agent de réticulation, A1-O-CO-R-CO-O-A1 en solution dans ledit solvant organique non miscible, ou, en variante, on forme une émulsion du type (H/E) par dispersion, sous agitation, d'une solution organique contenant ledit agent de réticulation dans une solution aqueuse de monomères et/ou oligomères de resvératrol, additionnée d'un agent alcalin en solution aqueuse pour ajuster le pH de la phase dispersante à 9-11,5 environ.

On utilise les agents émulsionnants à raison de 2 à 15 % en poids environ, par rapport au poids de la dispersion, notamment de 3 à 8 % environ.

Des agents appropriés correspondent à ceux habituellement utilisés, tels que ceux commercialisés sous la marque Span® (alcools hexyliques estérifiés) ou Tween® (esters d'acides gras et de sorbitol avec de l'oxyde d'éthylène).

Selon les quantités relatives des phases aqueuses et organiques et de l'émulsionnant, on forme une émulsion du type H/E ou E/H.

On réalise l'agitation de manière à homogénéiser rapidement les solutions aqueuses et organiques, par exemple en utilisant un barreau magnétique à 500-1000 tours/min. ou une hélice à 800-2000 tours/min. La durée de cette étape est généralement de l'ordre de 30 minutes.

La réticulation se produit à l'interface des gouttelettes de l'émulsion.

On récupère les esters réticulés formés, par exemple par centrifugation. Les produits lavés et séchés se présentent sous forme de poudre fluide.

Les esters formés peuvent être également récupérés par dilution du mélange réactionnel à l'aide d'un ou plusieurs solvants, décantatation et/ou centrifugation, et lavage.

L'observation au microscope montre que les gouttelettes se présentent sous forme de particules sensiblement sphériques, de taille homogène.

Leur diamètre peut varier de 25 à 300 µm environ selon les conditions mises en oeuvre pour leur obtention.

Dans le cas de microcapsules renfermant des principes actifs, on ajoute ces derniers de préférence dans la phase aqueuse ou organique dans laquelle ils sont solubles.

Les monomères et/ou oligomères de resvératrol, ORs en abrégé, mis en oeuvre dans l'étape d'estérification peuvent être obtenus à partir de diverses sources végétales. On citera les Vitacées, Umbellifères, Myrtacées, Diptérocarpacées, Cypéracées, Gnétacées, Légumineuses, Graminées, Séricées, Haemodoracées, Musacées, Polygonacées, Pinacées, Cupressacées, Césalpiniacées, Poacées, et Solanacées.

De manière avantageuse, on utilise des monomères et/ou oligomères de resvératrol tels qu'obtenus par extraction, à l'aide d'eau et/ou d'un solvant organique, à partir de rafles de vigne.

Le procédé d'obtention des monomères et, des oligomères de resvératrol, qui est également utilisé par l'invention, comprend les étapes
- d'extraction par addition, à des rafles de vigne, d'eau et/ou de solvant(s) organiques(s), en soumettant le tout à un traitement tel que macération/lixiviation, ultrasons ou micro-ondes,
- de délipidation avant ou après l'étape d'extraction à l'aide d'un solvant de type éther de pétrole, hexane ou chloroforme,
- extraction supplémentaire de l'extrait récupéré par un solvant organique du type acétate d'éthyle ou éther éthylique,
- de concentration de l'extrait brut obtenu, et, si souhaité, de sa lyophilisation.

Le pourcentage de resvératrol et d'oligomères dans l'extrait brut dépend étroitement du cépage utilisé et, pour un cépage donné de la méthode d'extraction et des solvants mis en oeuvre.

Selon une disposition particulièrement intéressante, compte tenu de l'enrichissement en ORs qu'elle permet d'atteindre, on soumet l'extrait brut à une étape de purification par chromatographie. Une technique spécialement satisfaisante correspond à la chromatographie de partage centrifuge (CPC). Cette technique est notamment décrite par A.P. FOUCAULT, Ed., Centrifugal Partition Chromatography, Chromatographic Science Series, Marcel Dekker Inc., 1995, 68, ou W.D. CONWAY, Ed., Countercurrent Chromatography apparatus theory and applications, VCH Publishers Inc., 1990.

La CPC est basée sur le partage des solutés entre deux phases liquides non miscibles préparées par mélange de deux ou plusieurs solvants ou solutions. L'une des deux phases est maintenue stationnaire par une force centrifuge.

Les solvants, leurs proportions et le débit choisis dépendent étroitement à la fois de la stabilité de la phase stationnaire au sein de la colonne de CPC et de la pression réelle.

Des résultats performants ont été obtenus avec le mélange hexane/acétate d'éthyle/éthanol/eau avec par exemple des proportions respectives de 6/48/11/42 ou 4/5/3/3.

Il est également possible cependant de ne pas utiliser d'hexane ou de remplacer au moins l'un desdits solvants par un solvant équivalent à condition d'en modifier les proportions.

Ansi, l'hexane peut être remplacé par des carbures saturés, voire insaturés, apolaires et non miscibles à l'eau, comme par exemple l'heptane, le cyclohexane, ou encore des solvants chlorés comme le chloroforme.

De même, on pourra utiliser à la place de l'acétate d'éthyle, des solvants carbonylés ou carboxylés, tels que l'acétone, la méthyléthylcétone, la méthylisobutylcétone, la méthylterbutylcétone.

D'autres alcools que l'éthanol pourront également être mis en oeuvre dans le mélange défini ci-dessus, comme par exemple, le méthanol, le n-propanol, le propan-2-ol, le n-butanol, le butan-2-ol.

L'eau peut être remplacée, au moins en partie, voire en totalité, par l'acétonitrile.

L'homme du métier choisira ainsi le ou les solvants les plus appropriés selon la nature de l'extrait purifié soumis à la CPC.

Grâce à l'invention, on dispose ainsi d'extraits bruts et de fractions enrichies comportant, comme constituants majoritaires, du resvératrol et/ou des oligomères de ce dernier, comme le montrent les chromatogrammes auxquels il est fait référence dans les exemples. Ces différents extraits, à savoir bruts ou enrichis entrent également en tant que tels dans le cadre de l'invention.

La mise en oeuvre d'étapes de séparations supplémentaires de CPC permet d'isoler de ces extraits enrichis le resvératrol monomère d'une part, et le resvératrol sous forme oligomères d'autre part. Ces séparations peuvent être effectuées sur les fractions enrichies à partir d'un extrait brut ou sur l'extrait brut lui-même en utilisant des mélanges de solvants appropriés selon les proportions convenant pour la séparation recherchée.

La dérivatisation des ORs conformément à l'invention permet de disposer de produits de grand intérêt dans de nombreux domaines.

La présence des groupes esters introduits confère une stabilité à l'air et à la lumière aux structures de resvératrol. De manière avantageuse, ces groupes sont éliminables uniquement lorsqu'ils sont placés dans les conditions où ces compositions doivent agir, ce qui permet d'exploiter les propriétés, notamment anti-radicalaires et anti-oxydantes du resvératrol, dans des conditions optimales.

L'innocuité des dérivés de l'invention les rend particulièrement intéressants pour toutes les applications impliquant une administration ou un usage par l'homme ou l'animal.

L'invention vise donc l'utilisation des compositions d'esters définies ci-dessus pour la fabrication de médicaments.

Ces médicaments peuvent également contenir d'autres principes actifs, en particulier des produits à effet protecteur vis-à-vis des réactions d'oxydation. On citera par exemple le β carotène ou la vitamine E.

Les préparations pharmaceutiques fabriquées selon l'invention sont utilisables notamment dans des traitements anti-tumoraux ou vaso-protecteurs.

Comme formes d'administration, on a recours à des formes appropriées pour la voie orale, comme les pilules, tablettes, gélules, ou gouttes. Ces préparations renferment avantageusement environ 50 à 200 mg d'équivalent de composition par unité de prise, de préférence environ 100 à 150 mg.

D'autres formes galéniques sont réalisées pour une administration par voie cutanée, sous-cutanée, intradermique, intramusculaire ou intraveineuse, notamment de gels, solutions et autres.

Les compositions de l'invention sont également utilisables avec avantage pour l'élaboration de préparations cosmétiques.

Ces préparations sont caractérisées en ce qu'elles renferment également au moins une composition de l'invention en une proportion permettant de disposer d'une quantité efficace d'ORs, et comprenant en association les excipients permettant leur application.

Les propriétés liposolubles conférées à ces préparations par la présence des groupes esters permettent de les incorporer aisément aux produits utilisés classiquement en cosmétique.

Les préparations de l'invention se présentent sous forme de crème, pommade, émulsion, gel, liposomes, lotion. Elles renferment environ de 0,2 à 5 % de produit actif.

Les compositions de l'invention sont également utilisables dans le domaine alimentaire. Les propriétés anti-radicalaires des ORs qu'elles renferment assurent une meilleure conservation des aliments.

Elles sont utilisables en tant qu'additifs pour divers produits tels que boissons et produits laitiers.

On peut également les utiliser sous forme de pâtes, de granulés ou de gels dans diverses confiseries.

Dans ces différentes applications, les compositions réticulées de l'invention sont en outre utilisables comme vecteurs de principes actifs. Ceux-ci sont retenus dans la masse spongieuse des compositions réticulées ou sont contenus dans les microcapsules.

Les microcapsules peuvent renfermer des produits actifs en thérapeutique humaine ou animale, ou utilisables dans le domaine alimentaire, notamment en diététique.

L'encapsulation permet de se libérer du caractère liposoluble ou hydrosoluble du produit, et de divers inconvénients, qu'il peut présenter pour les applications envisagées. Elle facilite également l'accès aux sites d'action et permet d'administrer des principes actifs qui jusqu'à présent soulevaient des problèmes à cet égard et/ou de les protéger provisoirement jusqu'à leur arrivée au site d'action.

L'invention sera illustrée ci-après par des exemples de préparation d'esters d'ORs et d'utilisation pour l'élaboration de médicaments et de préparations cosmétiques.

Dans ces exemples, il est fait référence aux figures 1 à 5 qui représentent
- les figures 1 à 4, les profils de chromatographie liquide haute performance (CLHP) d'extraits d'ORs selon l'invention,
- la figure 5 le spectre RMN ¹H du perhexanoate de resvératrol.

### Exemple 1 : Extraction d'ORs à partir de rafles de vigne

On met en oeuvre les protocoles suivants :

### - extraction par traitement aux ultrasons

On recueille les rafles de vigne correspondant à un cépage donné et, après les avoir lavées et séchées, on les soumet à une étape de broyage.

On ajoute à 100 g de broyat de rafles, 400 à 1000 ml, par exemple 800 ml, d'eau distillée, et/ou d'un ou plusieurs solvants organiques.

On utilise, par exemple : eau ; eau/acétone : 3/2 ou 1/1 ; méthanol ; éthanol ; eau/éthanol: 1/1 ; eau/acétate d'éthyle: 1/1 ; éthanol/acétone : 1/1 ; eau/éthanol/acétone: 2/1/1, 1/2/1 ou 1/1/2.

On soumet l'ensemble aux ultrasons, en opérant, le plus généralement pendant environ 30 min. à 3 h.

On filtre ensuite le mélange, puis on le concentre.

Une délipidation est effectuée avant ou après cette étape d'extraction. A cet effet, on extrait par un solvant tel que l'éther de pétrole, l'hexane ou le chloroforme.

Cet extrait est à son tour soumis à au moins une autre étape d'extraction. On utilise de l'acétate d'éthyle ou de l'éther éthylique à raison de 3 à 5 fois 100 ml.

Le produit obtenu est ensuite concentré, repris par de l'eau, lyophilisé et conservé sous forme de poudre. On dispose ainsi d'un extrait brut d'ORs.

On vérifie l'activité anti-radicalaire et anti-oxydante des extraits obtenus aux fins de sélection des conditions opératoires.

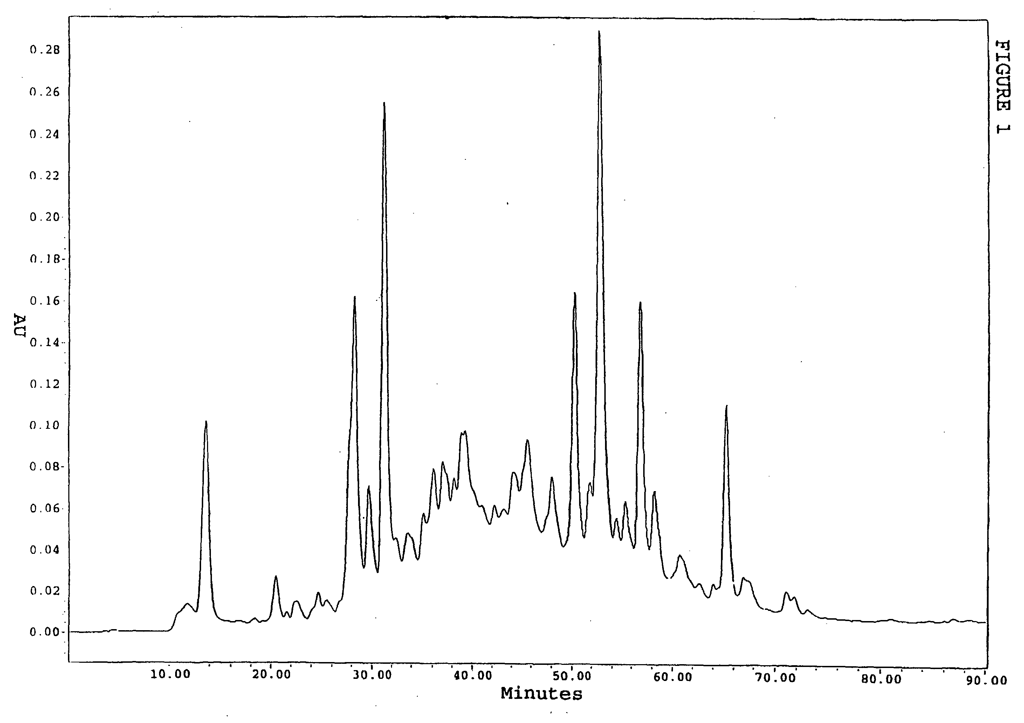

Sur la figure 1, on a représenté le chromatogramme en CLHP d'un extrait acétate d'éthyle obtenu, après traitement de 2 h aux ultrasons, de 100 g de rafles broyées (cépage Merlot) dans 800 ml d'eau/acétone: 3/2, et délipidation par l'éther de pétrole. L'élution a été réalisée avec A: H₂O/TFA ; 100/0,0025 (TFA = acide trifluoroacétique) et B : MeOH/TFA 100/0,0025, selon le gradient

| **min.** | **A** | **B** |
|---|---|---|
| **0** | 100 | 0 |
| **120** | 0 | 100 |

### - extraction par traitement aux micro-ondes (800 à 900 W maximum)

En variante, on opère comme indiqué ci-dessus, mais en remplaçant le traitement aux ultrasons par celui aux micro-ondes.

### - extraction par macération-lixiviation

On procède à une délipidation, dans les conditions données ci-dessus, avant ou après l'étape de macération-lixiviation.

Dans une colonne ouverte, on introduit 100 g de broyat de rafles de vigne, préparé comme indiqué ci-dessus, puis environ 1 à 2 l de solvant.

On utilise des mélanges eau distillée/solvants organiques, comme par exemple le mélange eau/acétone : 3/2. On laisse le mélange broyat/solvant durant 10 à 20 h puis on récupère l'extrait que l'on concentre jusqu'à 100 ml dans le cas d'un extrait contenant au moins 100 ml d'eau, ou à sec s'il s'agit d'un extrait contenant exclusivement un ou plusieurs solvants organiques, le résidu étant repris par 100 ml d'eau.

Une délipidation est effectuée avant ou après cette étape d'extraction comme décrit précédemment.

L'extrait obtenu subit ensuite une étape d'extraction avec de l'acétate d'éthyle ou de l'éther éthylique comme indiqué précédemment pour la préparation d'extraits par ultrasons ou micro-ondes.

Les rendements en extraits bruts préparés sont le plus généralement supérieurs à 0,5% du poids sec des rafles de départ, et peuvent atteindre 1 à 1,5% selon les conditions d'extraction utilisées et l'origine des rafles.

### Exemple 2 : Obtention de fractions enrichies en ORs par CPC

La figure 1 montre le chromatogramme CLHP d'un extrait brut, tel qu'obtenu selon l'exemple 1, contenant majoritairement du resvératrol et de la catéchine en proportions pratiquement équivalentes.

1 g de cet extrait, dissous dans 4 ml de phase stationnaire, est injecté en CPC.

On utilise un appareil SANKI modèle LLB-M fabriqué et commercialisé par la société Sanki Engineering (Kyoto, Japan).

### Principales caractéristiques de cet appareil :

- Vitesse de rotation : 0-2000 tours/min
- Capacité de la colonne : 230 ml
- Pression maximale : 60 x 10⁵ Pa.
- Matériau de rotor : poly-phénylènesulfure (PPS)
- Disque de partition : série de disques
- Cellule de partition : 2136
- Longueur des cellules : 15 mm.

Les phases stationnaire et mobile sont respectivement les phases inférieure et supérieure récupérées après agitation et décantation du mélange hexane/acétate d'éthyle/éthanol/eau : 6/48/11/42, étant donné la mise en oeuvre de la technique en mode ascendant dans cet exemple.

La vitesse de rotation est fixée à 1100 tours/min, le débit à 2 ml/min et la pression est de 34 X 10⁵ Pa.

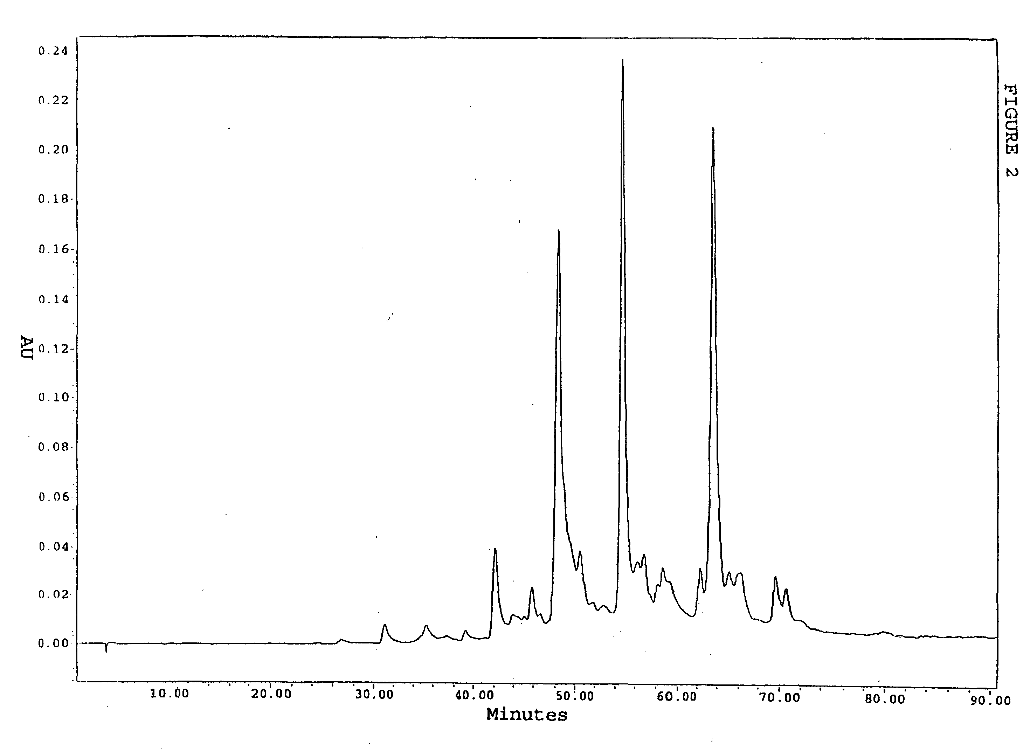

Dans ces conditions, la fraction enrichie en resvératrol et oligomères est recueillie rapidement, puisque les ORs sont élués dans les 30 premières minutes. La figure 2 représente le chromatogramme CLHP enregistré à partir de cette fraction enrichie en ORs. On récupère ainsi 120 mg d'ORs, soit un enrichissement de l'ordre de 8.

### Exemple 3 : Obtention de resvératrol monomère d'une part et des formes oligomères d'autre part par CPC.

Les 120 mg d'ORs récupérés à l'exemple 2, dissous dans 2 ml de phase stationnaire, sont séparés en mode ascendant. La phase stationnaire constitue la phase inférieure du mélange hexane/acétate d'éthyle/éthanol/eau 4/5/3/3 et la phase mobile la phase supérieure de ce même mélange. La vitesse de rotation est de 1100 tours/min, le débit de 2 ml/min et la pression de 44 x 10⁵ Pa. Dans ces conditions, la fraction enrichie en revératrol est recueillie après 150 min d'élution et celle enrichie en oligomères de resvératrol après 240 min.

Les enrichissements des fractions de resvératrol monomère d'une part (7 mg) et d'oligomères d'autre part (20,5 mg) sont alors respectivement de l'ordre de 17 et 6. On en déduit un nouvel enrichissement en ORs de l'ordre de 4,5.

L'enrichissement par rapport à l'extrait brut de départ est ainsi d'environ 36.

L'analyse CLHP montre un pourcentage de resvératrol de l'ordre de 81 % (t_{R} = 55,6 min), soit 5,7 mg de resvératrol pur, ainsi que les pourcentages en oligomères de resvératrol de l'ordre de 31 % (t_{R} = 48,5 min) et 53 % (t_{R} = 63,8 min), soit 17,2 mg d'oligomères purifiés.

### Exemple 4 : En appliquant directement la méthode décrite à l'exemple 3 (durée de 4 h) à l'extrait brut de départ utilisé à l'exemple 2, on parvient à purifier le resvératrol (8 mg) et les oligomères de resvératrol (30 mg) de la même façon.

Les résultats, issus des chromatrogrammes CLHP sont satisfaisants. En effet, on obtient 2 fractions, l'une très enrichie en resvératrol (t_{R} = 56,1 min) avec un pourcentage de 74,0, l'autre très enrichie en oligomères de resvératrol (t_{R} = 48,9 min et t_{R} = 64,2 min) avec un pourcentage de 65,6.

### Exemple 5 : Variante d'obtention de fractions enrichies en ORs par CPC telles qu'obtenues dans l'exemple 1, à partir d'un extrait brut contenant majoritairement du resvératrol.

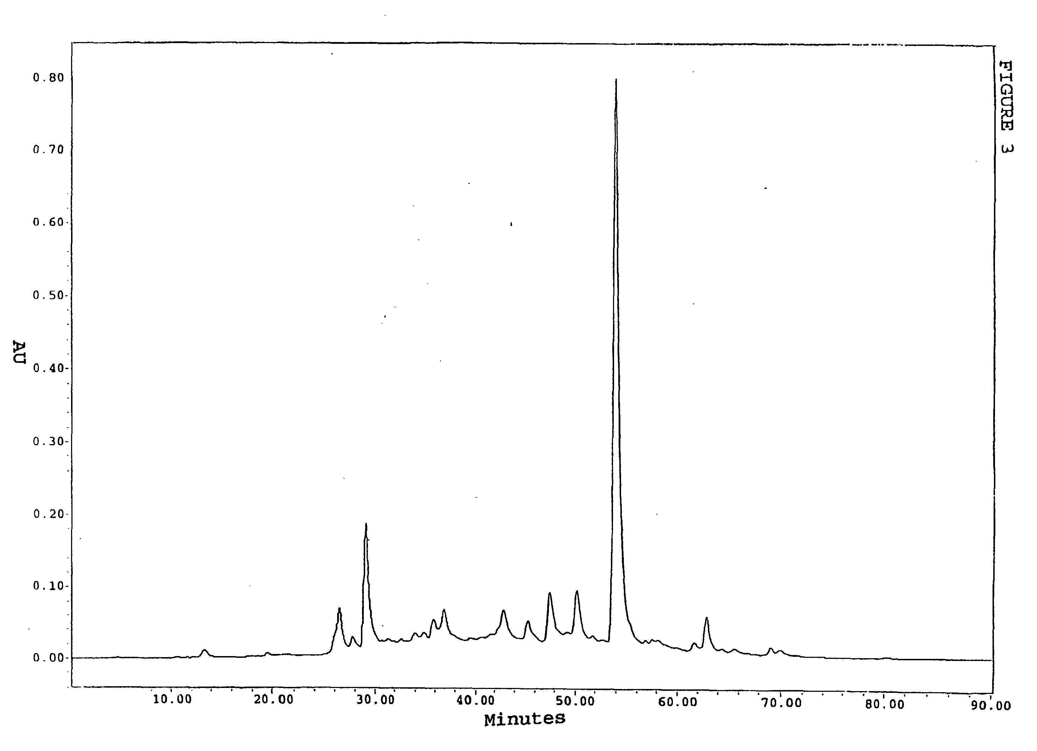

La figure 3 représente le chromatogramme CLHP de l'extrait brut d'ORs avant son étude par CPC.

Les conditions de séparation sont identiques à celles décrites dans l'exemple 2.

A partir d'1 g d'extrait brut de départ, 200 mg d'ORs enrichis sont recueillis au bout de 40 min, soit un facteur d'enrichissement égal à 5.

L'analyse CLHP de la fraction enrichie correspondante montre un pourcentage en resvératrol

(t_{R} = 54,6 min) de 88 %, soit 176 mg de resvératrol pur (facteur d'enrichissement de l'ordre de 6).

### Exemple 6 : Le fractionnement du même extrait brut de départ (exemple 5), selon le protocole expérimental utilisé dans l'exemple 3 précédent, permet d'obtenir un meilleur enrichissement en resvératrol pur.

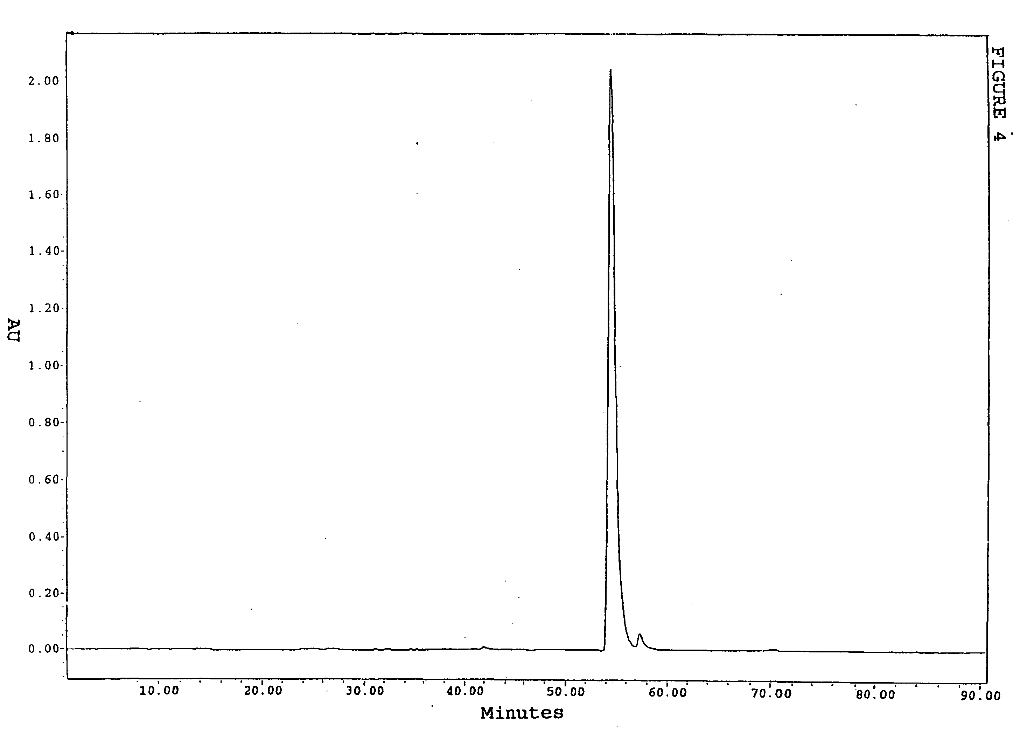

En effet, le chromatogramme CLHP (figure 4) montre une pureté du resvératrol ainsi obtenu de 99 % (t_{R} = 54,4 min).

### Exemple 7 : Synthèse de perhexanoate de resvératrol

A 15 mg de resvératrol (6,58 x 10⁻⁵ mole) en solution dans 2,5 ml de pyridine est ajouté, sous agitation et goutte à goutte, 0,09 ml de chlorure d'hexanoyle (88 mg ; 6,58 x 10⁻⁴ mole ; 10 éq.)

Le milieu réactionnel est agité à température ambiante, à l'abri de l'air (sous un léger flux d'azote) et de la lumière, pendant 12 heures.

Après concentration sous pression réduite, le résidu est repris par 20 ml de chloroforme.

La phase organique est ensuite lavée avec deux fois 50 ml d'une solution 0,1 M d'HCl, deux fois 50 ml d'eau distillée, deux fois 50 ml d'une solution de Na₂CO₃, puis deux fois 50 ml d'eau distillée.

Cette phase est séchée sur Na₂SO₄ anhydre, filtrée sur verre fritté n°4, concentrée à l'évaporateur rotatif et purifiée par chromatographie préparative sur couche épaisse de silice.

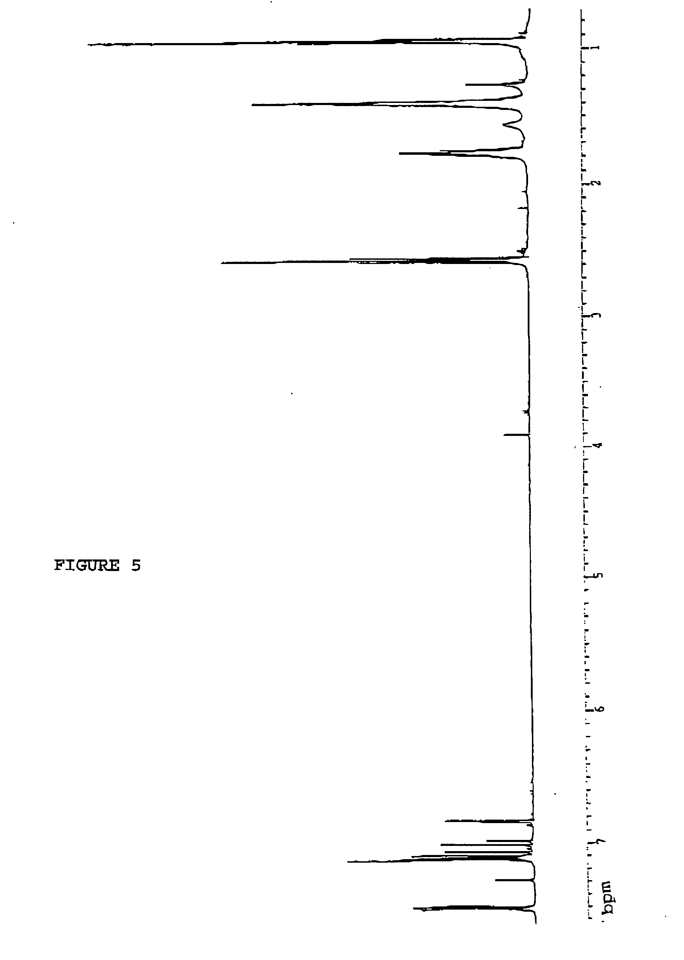

La figure 5 représente le spectre RMN ¹H.

Les analyses des spectres IR, RMN ¹H, et ¹³C confirment l'obtention de l'ester recherché.

### Exemple 8 : Synthèse du perpalmitate d'ORs

Un extrait lyophilisé d'ORs (30 mg ; 1,32 x 10⁻⁴ mole) préparé selon l'exemple 1, est dissous dans 5 ml de pyridine. A cette solution est ajouté goutte à goutte 0,40 ml de chlorure de palmitoyle (0,36 g ; 1,32 x 10⁻³ mole ; 10 éq.-resvératrol).

Le mélange réactionnel est laissé sous agitation à 70°C, sous un léger flux d'azote et à l'abri de la lumière, durant 3 heures.

Après concentration sous pression réduite, le résidu est traité comme dans l'exemple 7. Le produit ainsi obtenu est contrôlé par spectrométrie.

### Exemple 9 : Synthèse du perhexanoate d'ORs

Le protocole décrit à l'exemple 8 est appliqué en utilisant 200 mg d'extrait lyophilisé d'ORs (8,77 x 10⁻⁴ mole) et 1,23 ml de chlorure d'hexanoyle (1,18 g ; 8,77 x 10⁻³ mole ; 10 éq.-resvératrol)

Une analyse par spectrométrie IR et RMN ¹H confirme l'obtention de l'ester recherché.

### Exemple 10 : Synthèse du perbutyrate d'ORs

A 250 mg d'extrait lyophilisé d'ORs préparé selon l'exemple 1 (1,10 x 10⁻³ mole), dissous dans 5 ml d'eau distillée, sont additionnés sous agitation vive 25 ml de dichlorométhane.

Puis sont ajoutés 25 ml d'une solution aqueuse tamponnée d'hydrogénophosphate de sodium à 5% (pH # 10), 37 mg d'hydrogénosulfate de tétra-butylammonium
(1,10 x 10⁻⁴ mole ; 1/10 éq.-resvératrol) et 0,57 ml de chlorure de butyryle (0,58 g ; 5,48 x 10⁻³ mole ; 5 éq.-resvératrol).

Le mélange réactionnel est laissé 45 minutes sous agitation vive.

A la fin de la réaction, la phase organique est récupérée et lavée avec deux fois 15 ml d'eau distillée, puis évaporée sous pression réduite et purifiée par chromatographie préparative sur couche épaisse de silice.

Le produit ainsi obtenu est contrôlé par spectrométrie.

### Exemple 11 : Synthèse du perlaurate d'ORs

A un extrait lyophilisé d'ORs (250 mg ; 1,10 x 10⁻³ mole), préparé selon l'exemple 1, sont ajoutés 1,32 g d'acide laurique (6,58 x 10⁻³ mole ; 6 éq.-resvératrol) en solution dans 20 ml de 1,2-dichloroéthane.

Puis 1,36 g de dicyclocarbodiimide (DCC) (6,58 x 10⁻³ mole ; 6 éq.-resvératrol) sont dissous dans 5 ml de 1,2-dichloroéthane et ainsi ajoutés à la solution précédemment préparée. On ajoute ensuite 97 mg de 4-pyrrolidinopyridine (6,58 x 10⁻⁴ mole ; 6/10 éq.-resvératrol) dissous dans 1 ml de 1,2-dichloroéthane.

Le mélange réactionnel est laissé sous agitation pendant 2 heures, à température ambiante, sous un léger flux d'azote et à l'abri de la lumière.

La phase organique est ensuite filtrée et concentrée sous pression réduite.

Le résidu est alors repris par 25 ml d'hexane. La solution hexanique est filtrée, puis lavée avec deux fois 50 ml d'une solution de soude 0,1 M, puis avec deux fois 50 ml d'eau distillée. La phase organique est alors concentrée à l'évaporateur rotatif.

Le produit ainsi obtenu est analysé par spectrométrie.

### Exemple 12 : Synthèse du persorbate d'ORs

Le protocole décrit à l'exemple 11 est appliqué en utilisant 250 mg d'extrait lyophilisé d'ORs (1,10 x 10⁻³ mole) et 0,74 g d'acide sorbique (6,58 x 10⁻³ mole ; 6 éq.-resvératrol) et du chloroforme comme solvant organique.

### Exemple 13 : Préparation de microcapsules d'ORs réticulés par le chlorure de téréphtaloyle

Un extrait lyophilisé d'ORs (50 mg ; 2,19 x 10⁻⁴ mole), préparé selon l'exemple 1, est dissous dans 5 ml d'une solution d'hydrogénophosphate de sodium à 5% (pH # 10).

Cette solution est émulsionnée dans 20 ml d'une solution chloroformique à 5% de trioléate de sorbitane (Span 85®) par agitation à 3000 t/min. durant 5 minutes.

L'agent réticulant, le chlorure de téréphtaloyle (89 mg ; 4,39 x 10⁻⁴ mole ; 2 éq.-resvératrol), en solution dans 15 ml de chloroforme, est ensuite ajouté à l'émulsion.

L'agitation est maintenue pendant 30 minutes. A la fin de la réaction, le mélange réactionnel est centrifugé.

L'interface solide est récupérée, remise en suspension dans 30 ml de chloroforme et centrifugée de nouveau.

L'opération est renouvelée une fois avec du chloroforme et deux fois avec de l'eau distillée. Le culot de centrifugation est récupéré et remis en suspension dans 5 ml d'eau distillée. Les microcapsules ainsi préparées sont séchées par lyophilisation et contrôlées par microscopie optique.

### Exemple 14: Préparation de microcapsules d'ORs réticulés par le chlorure de sébacoyle

Le protocole décrit à l'exemple 13 est appliqué en utilisant 50 mg d'extrait lyophilisé d'ORs (2,19 x 10⁻⁴ mole) et 0,09 ml de chlorure de sébacoyle (0,10 g ; 4,38 x 10⁻⁴ mole ; 2 éq.-resvératrol).

### Exemple 15: Préparation de microcapsules d'ORs réticulés par le chlorure d'adipoyle

Le protocole décrit à l'exemple 13 est appliqué en utilisant 50 mg d'extrait lyophilisé d'ORs (2,19 x 10⁻⁴ mole) et 0,06 ml de chlorure d'adipoyle (80 mg ; 4,38 x 10⁻⁴ mole ; 2 éq.-resvératrol).

### Exemple 16 : Préparation cosmétique anti-solaire

Une émulsion anti-solaire à propriétés antivieillissement cutané est réalisée en mélangeant un filtre solaire avec un ester préparé selon l'invention et des excipients pour crème.

Exemple de formulation :

| | |
|---|---|
| Néo Héliopan E 1000^{R} (isopropylméthoxycinnamate et éthyldiisopropylcinnamate) | 3 % |
| Perlaurate d'ORs selon l'exemple 6 | 3 % |
| Excipients pour crème E/H | qs |

Composition d'excipients :
- Propylène glycol dicaprylate/dicarate + stéaralkonium hectorite + propylène carbonate (Miglyol 840 gel B®) 20,0 %
- Bis-diglycéryl caprylate/caprate/isostéarate/hydroxystéarate adipate (Softisan 649®) 5,0 %
- Isostéaryl diglycéryl succinate (Imwitor) 780 K® 5,0 %
- Huile de paraffine 8,0 %
- Paraffine solide 3,0 %
- Sulfate de magnésium 2,0 %
- Eau qsp 100 %

### Exemple 17 : Préparation de gélules pour utilisation en diététique

Du perlaurate d'ORs préparé selon l'exemple 11 est mélangé à du sélénium et de la vitamine E ;
- Perlaurate d'ORs : 85 mg (correspondant à 25 mg d'ORs),
- Acétate du DL- α-tocophérol 40 mg,
- Sélénium : 50 mg

### Exemple 18 : Préparation de médicament veinotonique et vasculoprotecteur

Des gélules sont préparées à partir de 230 mg de perhexanoate d'ORs (correspondant à 100 mg d'ORs), préparés selon l'exemple 9, et d'excipients pour un enrobage gastro-résistant, comme l'acétophtalate de cellulose.

### Exemple 19 : Préparation d'un gel buccal utilisable en radiothérapie

On formule la composition suivante :
- Gel de Carbopol ^{R} 934 P à 2 % 89,85 g
- Para hydroxybenzoate de méthyle sodé 0,13 g
- Para hydroxybenzoate de propyle sodé 0,02 g
- Labrafil ^{R} 5 g
- Perhexanoate d'ORs préparé selon l'exemple 9 5 g
   (correspondant à 2,2 g d'ORs)

## Revendications

1. Compositions à base de dérivés du resvératrol **caractérisées en ce qu'**il s'agit essentiellement d'esters de resvératrol, monomères et/ou oligomères, les monomères comportant au moins un groupe ester de formule -O-CO-A, et les oligomères étant formés de motifs monomères réunis par des liaisons carbone-carbone, ou éther, et/ou de monomères réticulés par des groupes -O-CO-R-CO-O-,
- A représentant un radical alkyle d'au moins deux atomes de carbone, linéaire ou ramifié, saturé ou insaturé, un radical aryle, excepté phényle dans le cas d'une composition de resvératrol monomère, aralkyle ou aralkylène, et
- R représentant un radical alkylène de 0 à 10 atomes de carbone, saturé ou insaturé, et/ou 1 radical arylène ayant 1 à 3 cycles et/ou un radical hétérocyclique, et les diastéréoisomères de ces motifs.

2. Compositions selon la revendication 1, **caractérisées en ce qu'**elles sont essentiellement à base d'esters monomères et/ou oligomères comportant au moins un groupe -O-CO-A.

3. Compositions selon la revendication 2, **caractérisées en ce que** A représente un radical d'acide gras saturé ou insaturé, par exemple d'acide butyrique ; valérique, hexanoïque, sorbique, laurique; palmitique; stéarique, oléique, linoléique; linolénique, α linolénique, arachidonique, éicosapentaénoique; et docosahexaénoïque.

4. Compositions selon la revendication 1, **caractérisées en ce qu'**elles sont essentiellement à base de monomères et/ou d'oligomères réticulés par l'intermédiaire de ponts -CO-R-CO où R représente un radical alkylène de 0 à 10 atomes de carbone, saturé ou insaturé, et/ou un radical arylène comportant 1 à 3 cycles et/ou un radical hétérocyclique.

5. Composition selon la revendication 4, **caractérisée en ce que** R est un radical d'un diacide choisi parmi l'acide malique, malonique, glutarique, phtalique, d'un chlorure de diacides, comme le dichlorure de téréphtaloyle, le dichlorure de succinyle, le dichlorure de sébacoyle, et le dichlorure d'adipoyle, d'un anhydride, ou encore d'un isocyanate comme le diisocyanate de toluène ou d'hexaméthylène.

6. Compositions selon l'une quelconque des revendications 4 ou 5, **caractérisées en ce qu'**elles se présentent sous forme de microcapsules.

7. Compositions selon l'une quelconque des revendications 4 ou 5, **caractérisées en ce qu'**elles se présentent sous forme de masse spongieuse.

8. Procédé d'obtention de compositions à base de dérivés de resvératrol, **caractérisé en ce qu'**il comprend la réaction de monomères et/ou d'oligomères de resvératrol avec comme agents d'acylation des composés de formule A-CO-O-A1, ou A1-O-CO-R-CO-O-A1, où
- A représente un radical alkyle d'au moins deux atomes de carbone, linéaire ou ramifié, saturé ou insaturé, un radical aryle, aralkyle ou aralkylène,
- R représente un radical alkylène de 0 à 10 atomes de carbone, saturé ou insaturé, et/ou 1 radical arylène ayant 1 à 3 cycles et/ou un radical hétérocyclique, et
- A1 représente un atome d'hydrogène, d'halogène, un radical alcoyle en C1 à C8, ou aryle, un groupe -CO-A, ou isocyanate, A et A1 ne pouvant représenter respectivement un radical phényle et un atome de chlore dans A-CO-O-A1.

9. Procédé selon la revendication 8, **caractérisé en ce que** l'estérification est réalisée selon la réaction de Schotten Baumann, en milieu aqueux alcalin.

10. Procédé selon la revendication 8 **caractérisé en ce que** lorsqu'on utilise des diacides ou leurs dérivés, on forme une émulsion de type (E/H) par dispersion, sous agitation, d'une solution aqueuse alcaline des monomères et/ou oligomères de resvératrol dans un solvant organique non miscible à l'eau, puis on ajoute l'agent de réticulation, A1-O-CO-R-CO-O-A1 en solution dans ledit solvant organique non miscible, ou, en variante, on forme une émulsion du type (H/E) par dispersion, sous agitation, d'une solution organique contenant ledit agent de réticulation dans une solution aqueuse de monomères et/ou oligomères de resvératrol, additionné d'un agent alcalin en solution aqueuse pour ajuster le pH de la phase dispersante à 9-11,5 environ.

11. Procédé selon l'une quelconque des revendications 8 à 10, **caractérisé en ce que** les monomères et/ou oligomères de resvératrol mis en oeuvre sont tels qu'obtenus par extraction à l'aide d'eau et/ou d'un solvant organique à partir de rafles de vigne.

12. Application des esters selon l'une des revendications 1 à 7, renfermant le cas échéant un ou plusieurs principes actifs, dans le domaine cosmétique ou en diététique.

13. Utilisation des esters selon l'une des revendications 1 à 7, renfermant le cas échéant un ou plusieurs principes actifs, pour préparer un médicament.

## Patentansprüche

1. Stoffkomposition auf der Grundlage von Resveratrol-Derivaten, **dadurch gekennzeichnet, daß** es sich im wesentlichen um monomere und/oder oligomere Resveratrol- ester handelt, wobei die monomeren mindestens eine Estergruppe der Formel -O-CO-A enthalten und die oligomeren aus Monomer-Einheiten gebildet sind, die durch C-C-Bindungen oder Etherbindungen und/oder durch Gruppen -O-CO-R-CO-Overnetzte Monomere gebildet sind, wobei
- A einen Alkylrest mit mindestens zwei C-Atomen, der gradkettig oder vernetzt, gesättigt oder ungesättigt ist, einen Arylrest, ausgenommen Phenyl im Fall einer Stoffkomposition von Resveratrol-Monomer, Aralkyl oder Aralkylen bedeutet, und
- R einen Alkylenrest mit 0 bis 10 C-Atomen, der gesättigt oder ungesättigt ist, und/oder einen Arylenrest mit 1 bis 3 Ringen und/oder einen heterocyclischen Rest bedeutet, und die Diasteromeren dieser Einheiten.

2. Komposition nach Anspruch 1, **dadurch gekennzeichnet, daß** sie im wesentlichen auf der Grundlage von monomeren und/oder oligomeren Estern mit mindestens einer Gruppe -O-CO-A vorliegen.

3. Komposition nach Anspruch 2, **dadurch gekennzeichnet, daß** A einen Rest einer gesättigten oder ungesättigten Fettsäure, beispielsweise Buttersäure, Valeriansäure, Hexansäure, Sorbinsäure, Laurinsäure, Palmitinsäure, Stearinsäure, Oleinsäure, Linolsäure, Linolensäure, α-Linolensäure, Arachidonsäure, Eicosapentaensäure und Docosahexaensäure bedeutet.

4. Komposition nach Anspruch 1, **dadurch gekennzeichnet, daß** ihre Grundlage im wesentlichen Monomere und/oder Oligomere sind, die mittels -CO-R-CO-vernetzt sind, worin R einen Alkylenrest mit 0 bis 10 C-Atomen, der gesättigt oder ungesättigt ist, und/oder einen Arylenrest mit 1 bis 3 Ringen und/oder einen heterocyclischen Rest bedeutet.

5. Komposition nach Anspruch 4, **dadurch gekennzeichnet, daß** R ein Rest einer Dicarbonsäure ist, die ausgewählt ist unter Äpfelsäure, Malonsäure, Glutarsäure, Phthalsäure, einem Chlorid von Dicarbonsäuren, wie das Dichlorid von Terephthalsäure, Bernsteinsäure, Sebacinsäure und Adipinsäure, einem Anhydrid oder auch einem Isocyanat, wie Toluol-diisocyanat oder Hexamethylen-diisocyanat.

6. Komposition nach einem der Ansprüche 4 oder 5, **dadurch gekennzeichnet, daß** sie in Form von Mikrokapseln vorliegen.

7. Komposition nach einem der Ansprüche 4 oder 5, **dadurch gekennzeichnet, daß** sie in Form einer schwammartigen Masse vorliegen.

8. Verfahren zur Herstellung von Stoffkompositionen auf der Grundlage von Resveratrolderivaten, **dadurch gekennzeichnet, daß** es die Umsetzung von Monomeren und/oder Oligomeren von Resveratrol mit Verbindungen der Formel A-CO-O-A1 oder A1-O-CO-R-CO-O-A1 als Acylierungsmittel umfaßt, wobei
- A einen Alkylrest mit mindestens zwei C-Atomen, der gradkettig oder verzweigt, gesättigt oder ungesättigt ist, einen Aryl-, Aralkyl- oder Aralkylenrest bedeutet,
- R einen Alkylenrest mit 0 bis 10 C-Atomen, der gesättigt oder ungesättigt ist, und/oder einen Arylenrest mit 1 bis 3 Ringen und/oder einen heterocyclischen Rest bedeutet, und
- A1 ein Wasserstoffatom, Halogenatom, einen C1- bis C8-Alkoylrest, oder Arylrest, eine Gruppe -CO-A oder Isocyanat bedeutet, wobei A und A1 in A-CO-O-A1 jeweils nicht einen Phenylrest und ein Chloratom bedeuten können.

9. Verfahren nach Anspruch 8, **dadurch gekennzeichnet, daß** die Veresterung nach der Schotten-Baumann-Reaktion in wässerig-alkalischem Milieu durchgeführt wird.

10. Verfahren nach Anspruch 8, **dadurch gekennzeichnet, daß** man bei Verwendung von Dicarbonsäuren oder deren Derivaten eine Emulsion vom Typ Wasser/Öl (E/H) bildet, indem man unter Rühren eine wässerig-alkalische Lösung von Monomeren und/oder Oligomeren von Resveratrol in einem mit Wasser nicht mischbaren organischen Lösungsmittel dispergiert und dann das Vernetzungsmittel A1-O-CO-R-CO-O-A1 in Lösung in dem nicht mischbaren organischen Lösungsmittel zufügt oder als Abwandlung eine Emulsion vom Typ Öl/Wasser (H/E) bildet, indem man unter Rühren eine organische Lösung, welche das Vernetzungsmittel enthält, in einer wässerigen Lösung von Monomeren und/oder Oligomeren von Resveratrol dispergiert, der ein alkalisches Mittel in wässeriger Lösung zugesetzt ist, um den pH der dispergierenden Phase auf etwa 9 bis 11,5 einzustellen.

11. Verfahren nach einem der Ansprüche 8 bis 10, **dadurch gekennzeichnet, daß** die eingesetzten Monomeren oder Oligomeren von Resveratrol solche sind, die aus Rebenstielen durch Extraktion mit Wasser und/oder einem organischen Lösungsmittel erhalten wurden.

12. Verwendung von Estern nach einem der Ansprüche 1 bis 7, welche gegebenenfalls ein oder mehrere aktive Prinzipien einschließen, im Gebiet der Kosmetik oder diätetischen Ernährung.

13. Verwendung von Estern nach einem der Ansprüche 1 bis 7, welche gegebenenfalls ein oder mehrere aktive Prinzipien umfassen, zur Herstellung eines Medikaments.

## Claims

1. Compositions based on derivatives of resveratrol **characterized in that** they are essentially based on esters of resveratrol, monomers and/or oligomers, the monomers comprising at least one ester group of formula -O-CO-A, and the oligomers being formed from monomer units joined by carbon-carbon bonds, or ether, and/or monomers cross-linked by -O-CO-R-CO-O- groups
- A representing an alkyl radical with at least two carbon atoms, linear or branched, saturated or unsaturated, an aryl radical, except for the phenyl radical in the case of a composition of resveratrol monomer, aralkyl or aralkylene, and
- R representing an alkylene radical with 0 to 10 carbon atoms, saturated or unsaturated, and/or 1 arylene radical having 1 to 3 rings and/or a heterocyclic radical, and the diastereoisomers of these units.

2. Compositions according to claim 1, **characterized in that** they are essentially based on monomer and/or oligomer esters comprising at least one -O-CO-A group.

3. Compositions according to claim 2, **characterized in that** A represents a saturated or unsaturated fatty acid radical, for example butyric, valeric, hexanoic, sorbic, lauric, palmitic, stearic, oleic, linoleic, linolenic, a linolenic, arachidonic, eicosapentaenoic, and docosahexaenoic acid.

4. Compositions according to claim 1, **characterized in that** they are essentially based on cross-linked monomers and/or oligomers using -CO-R-CO bridges where R represents an alkylene radical with 0 to 10 carbon atoms, saturated or unsaturated, and/or an arylene radical comprising 1 to 3 rings and/or a heterocyclic radical.

5. Composition according to claim 4, **characterized in that** R is a radical of a diacid chosen from the following: malic, malonic, glutaric, phthalic acids, a chloride of diacids, such as terephthaloyl dichloride, succinyl dichloride, sebacoyl dichloride, and adipoyl dichloride, an anhydride, or also an isocyanate such as toluene or hexamethylene diisocyanate.

6. Compositions according to any one of claims 4 or 5, **characterized in that** they are presented in the form of microcapsules.

7. Compositions according to any one of claims 4 or 5, **characterized in that** they are presented in the form of a spongy mass.

8. Process for obtaining compositions based on derivatives of resveratrol, **characterized in that** it comprises the reaction of monomers and/or of oligomers of resveratrol with as acylation agents the compounds of formula A-CO-O-A1, or A1-O-CO-R-CO-O-A1, where
- A represents an alkyl radical with at least two carbon atoms, linear or branched, saturated or unsaturated, an aryl, aralkyl or aralkylene radical,
- R represents an alkylene radical with 0 to 10 carbon atoms, saturated or unsaturated, and/or 1 arylene radical having 1 to 3 rings and/or a heterocyclic radical, and
- A1 represents a hydrogen atom, a halogen atom, a C1 to C8 alkyl radical, or aryl, a -CO-A group or isocyanate, A and A1 cannot respectively represent a phenyl radical and a chlorine atom in A-CO-O-A1.

9. Process according to claim 8, **characterized in that** the esterification is carried out according to the Schotten Baumann reaction, in alkaline aqueous medium.

10. Process according to claim 8 **characterized in that** when diacids or their derivatives are used, an emulsion of (W/O) type is formed by dispersion, under agitation, of an alkaline aqueous solution of the monomers and/or oligomers of resveratrol in an organic solvent which is not miscible with water, then the cross-linking agent, A1-O-CO-R-CO-O-A1 in solution in said non-miscible organic solvent is added, or, as a variant, an emulsion of (O/W) type is formed by dispersion, under agitation, of an organic solution containing said cross-linking agent in an aqueous solution of monomers and/or oligomers of resveratrol, with an alkaline agent in aqueous solution added to it to adjust the pH of the dispersing phase to approximately 9-11.5.

11. Process according to any one of claims 8 to 10, **characterized in that** the monomers and/or oligomers of resveratrol used are as those obtained by extraction using water and/or an organic solvent from vine stalks.

12. Use of the esters according to one of claims 1 to 7, containing, if appropriate, one or more active ingredients, in the field of cosmetics or dietetics.

13. Use of esters according to one of claims 1 to 7, containing, if appropriate, one more active ingredients, to prepare a medicament.
